# EUROPEAN PATENT SPECIFICATION

(11) **EP 2 965 746 B1**
(45) Date of publication and mention of the grant of the patent: **13.03.2019**
(21) Application number: 14002377.1
(22) Date of filing: 10.07.2014
(51) Int. Cl.: A61K 9/00, A61K 9/20

(54) **An oral pharmaceutical composition comprising ibuprofen, ibuprofen sodium dihydrate, pseudoephedrine hydrochloride and chlorpheniramine maleate**
Orale pharmazeutische Zusammensetzung mit Ibuprofen, Ibuprofennatriumdihydrat, Pseudoephedrinhydrochlorid und Chlorpheniraminmaleat
Composition pharmaceutique orale comprenant de l'ibuprofène, du dihydrate de sodium d'ibuprofène, de l'hydrochlorure de pseudoéphédrine et du maléate de chlorophéniramine

(43) Date of publication of application: 13.01.2016
(73) Proprietor: Santa Farma Ilaç Sanayi A.S., 34382 Istanbul (TR)
(72) Inventor: Kanik, Bayram, 34091 Istanbul (TR); Öztuna, Banu, 34382 Istanbul (TR); Aksoy, Ediz, 34091 Istanbul (TR); Ünver, Levent, 34091 Istanbul (TR); Öner, Levent, 06610 Ankara (TR)
(74) Representative: Bulut, Pinar

(56) References cited:
- WO-A1-2007/092784
- WO-A1-2010/056216

## Description

### FIELD OF INVENTION

The present invention relates to an oral pharmaceutical composition, it comprises;a. 200 mg ibuprofen and ibuprofen sodium dihydrate mixture;b. 30 mg pseudoephedrine hydrochloride;c. 2 mg chlorpheniramine maleate; and in an acceptable carrier, characterized in that the weight ratio of ibuprofen to ibuprofen sodium dihydrate as free acid equivalent is 7:1 to 1:1 and wherein 15% or more of total ibuprofen dissolves in 15 minutes in 900 ml of 0.1N HCl solution at pH 1.2 at 37°C using a USP paddle apparatus rotating at 50 RPM.

### BACKGROUND OF THE INVENTION

The addition of a NSAID to a composition containing an antihistamine and/or a decongestant has been shown to enhance the efficacy of the antihistamine and decongestant, thus permitting a reduction in the total dose of either or both. The combination of a NSAID with reduced levels of antihistamine and/or decongestant has been shown to provide greater symptomatic relief of rhinitis, including allergy, cold, cold-like, and flu symptoms, as conventional products containing higher amounts of antihistamine and decongestants.

Currently, there are many commercial products that incorporate a non-steroidal antiinflammatory drug (NSAID) together with an antihistamine and/or a decongestant into a single dosage unit. Advil® Cold & Sinus, Nurofen® Cold & Flu, Sudafed® Sinus Pressure & Pain are examples of commercial products each containing ibuprofen and pseudoephedrine hydrochloride, while Advil® Allergy & Sinus contains ibuprofen, pseudoephedrine hydrochloride, and chlorpheniramine maleate.

US 2004/0253311 A1 (Wyeth) discloses a multilayer pharmacutical composition comprising a NSAID, a decongestant, and an antihistamine preferably in a multi-layer tablet consisting an immediate release layer and an extended release layer.

EP 1572206 B1 (Wyeth) discloses pharmaceutical compositions for oral use comprising 200 to 400 mg of ibuprofen, 20 to 45 mg of pseudoephedrine hydrochloride, and 1 to 3 mg of chlorpheniramine maleate in a single unit dose, wherein the ratio of amount of pseudoephedrine or chlorpheniramine, or both, to ibuprofen relative to an approved dose of each, is less than or equal to 0.75:1.

WO 2010/056216 A1 (Berko İlaç) relates to liquid pharmaceutical compositions for oral administration comprising 4 to 200 mg/ml of ibuprofen, 0.6 to 30 mg/ml of pseudoephedrine hydrochloride, 0.04 to 2 mg/ml of chlorpheniramine maleate, and an alkaline salt or a mixture of alkaline salts in order to maintain the pH level of the final composition between around 7 and around 9.

TR 2012/06317 (Abdi Ibrahim) relates to pharmaceutical compositions for oral administration comprising pharmaceutically effective amount of ibuprofen or a pharmaceutically acceptable salt thereof, pharmaceutically effective amount of pseudoephedrine or a pharmaceutically acceptable salt thereof, pharmaceutically effective amount of chlorpheniramine or a pharmaceutically acceptable salt thereof in an acceptable carrier in the presence of basic agents.

EP 0418043 B1 (Nicholas Kiwi) relates to taste-masked, non-effervescent, water soluble sachet formulations which contain a potassium, sodium, arginine, or lysine salt of ibuprofen and a bicarbonate, hydrogen phosphate or tribasic citrate of an alkali metal. The disclosed formulations contain only about 20% by weight or less of ibuprofen salt and around 50% by weight or more of further auxiliary materials, in particular dextrose.

EP 0584108 B1 (Boots Company) relates to solid dosage forms comprising S(-)-sodium ibuprofen having an enantiomeric purity of at least 90%, together with a pharmaceutically acceptable carrier, with the exception of (a) compositions consisting of a solution of S(-)-sodium ibuprofen in water with no additional pharmaceutical excipient and (b) compositions comprising the calcium salt of S(+)-ibuprofen.

EP 0881899 B1 (Boots Company) relates to non-effervescent solid dosage forms comprising sodium salt of racemic ibuprofen. The dosage forms may also include other compatible pharmacologically active ingredients (e.g., codeine) and/or enhancing agents. The dosage forms may include any ingredient commonly used in a cough, cold or flu remedy (e.g., caffeine or another xanthine derivative), and/or another analgesic, and/or a skeletal muscle relaxant, and/or an antihistamine (e.g. chlorpheniramine), and/or a decongestant (e.g., pseudoephedrine), and/or a cough suppressant and/or an expectorant.

WO 94/10994 A1 (Boots Company) discloses an effervescent powder or tablet composition comprising sodium ibuprofen dihydrate in quantities of 6.0-14.5% by weight wherin the pH of an aqueous solution formed from 1 g of the composition in 100 ml of purified water is greater than 5.0.

EP 1410793 B1 (Bayer) discloses a non-effervescent tablet for oral administration of sodium ibuprofen wherein the tablet core consists of 50 to 100% by weight of sodium ibuprofen dihydrate.

WO 2011/005478 A2 (Wyeth) discloses a pharmaceutical composition in the form of a tablet or caplet comprising ibuprofen sodium wherein the composition has a sodium content of less than 23 mg/dosage unit.

Examples of commercial formulations containing ibuprofen sodium dihydrate include Fast-acting Advil® 256 mg Film-coated tablets (Pfizer Consumer Healthcare), Nurofen® Express 256 mg Caplets/Tablets (Reckitt Benckiser Healthcare), Saridon® N Film-coated tablets (Bayer AG). These formulations contain 256 mg of sodium ibuprofen dihydrate per tablet, that is equivalent to 200 mg of ibuprofen free acid. These formulations dissolve quickly in vitro, have the same extent of absorption as other fast-acting ibuprofen formulations (e.g. formulations comprising ibuprofen lysine or ibuprofen arginate), and are absorbed into plasma more rapidly than conventional ibuprofen free acid formulations.

A major issue is to improve the onset of action of ibuprofen, particularly in the treatment of pain. It is believed that rapid disintegration of a formulation releases the drug into the body quickly leading to a more rapid onset of therapeutic action compared with a standard dosage form. Accordingly, it is desired to produce a solid dosage form for oral administration adapted to disintegrate quickly in the gastro-intestinal tract.

In the pharmaceutical composition according to the present invention, mixture of ibuprofen and ibuprofen sodium dihydrate is used. None of the prior art discloses pharmaceutical combinations comprising ibuprofen, ibuprofen sodium dihydrate, pseudoepehdrine hydrochloride, and chlorpheniramine meleate in a single dosage form for oral administration.

Although Advil® Allergy & Sinus (Pfizer Consumer Healthcare) contains ibuprofen, pseudoephedrine hydrochloride, and chlorpheniramine maleate, currently there is no commercially available product on the market that comprises a mixture of ibuprofen with ibuprofen sodium dihydrate, together with pseudoephedrine hydrochloride and chlorpheniramine maleate.

The surprising effect of the use of mixture of ibuprofen and ibuprofen sodium dihydrate may be reflected by the following mechanism: Ibuprofen sodium dihydrate dissolves faster and more in dissolution medium mimicing gastric conditions (i.e. 0.1 N HCl solution at pH 1.2) compared to ibuprofen free acid, while both substances dissolve more in dissolution medium mimicing intestinal conditions (i.e., at pH 4.5 and 6.8). The presence of ibuprofen sodium dihydrate in the pharmaceutical composition according to the present invention enables fast onset of analgesia due to enhanced dissolution properties of ibuprofen sodium dihydrate at gastric conditions (i.e., at pH 1.2). Meanwhile, the presence of ibuprofen free acid enables prolonged analgesic effect as ibuprofen free acid becomes soluble at increased pH levels in the body (i.e., at pH 4.5 and 6.8).

The present invention also provides a pharmaceutical composition having a lower sodium content relative to other commercially available ibuprofen sodium dihydrate dosage forms, providing a maximum daily sodium content of less than 140 mg/day.

### DETAILED DESCRIPTION OF THE INVENTION

Pseudoephedrine hydrochloride (Formula I), a sympathomimetic amine, is a potent decongestant of the upper respiratory tract that is found in many over-the-counter (OTC) preparations, either as a single ingredient or more commonly in combination with antihistamines, and/or paracetamol (acetaminophen) or an NSAID.

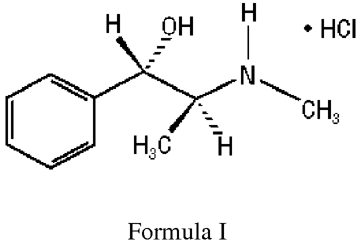

The usual adult dose of pseudoephedrine hydrochloride is 60 mg orally every 4-6 hours, up to a maximum of 240 mg per day. The usual pediatric dose of pseudoephedrine hydrochloride is 15 mg orally every 6 hours, up to a maximum of 60 mg per day for ages 2-5 and 30 mg every 6 hours, up to a maximum of 120 mg per day for ages 6-12.

Chlorpheniramine maleate (Formula II) is a first-generation alkylamine antihistamine used in the prevention of the symptoms of allergic conditions such as rhinitis and urticaria. Its sedative effects are relatively weak compared to other first-generation antihistamines.

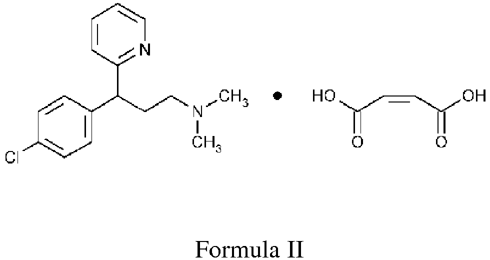

The usual adult dose of chlorpheniramine maleate is 4 mg every 4-6 hours, up to a maximum of 24 mg per day. The usual pediatric dose of chlorpheniramine maleate is 2 mg every 4-6 hours, up to a maximum of 12 mg per day.

Ibuprofen [(±)-(R,S)-2-(4-isobutylphenyl)-propionic acid] as depicted in Formula III, is a well-known, widely used and well-tolerated NSAID that has analgesic and antipyretic properties.

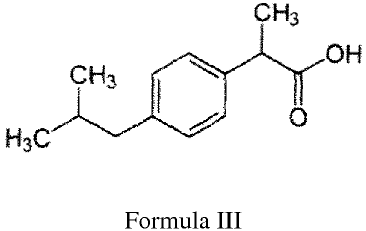

Pharmacokinetic studies have demonstrated that there is a linear dose-response relationship between the amount of drug administered and the area under the curve (AUC) following single doses of ibuprofen (200-800 mg).

With most analgesics, including ibuprofen, there is a significant correlation between plasma levels and the resultant degree of pain relief following oral administration. Both peak plasma concentrations (Cₘₐₓ) and maximal analgesic onset are achieved within 1.5-2 hours after oral administration of standard ibuprofen formulations. This delay may sometimes be disadvantageous, as patients expecting a fast onset of the analgesic effect may tend to unnecessarily raise the dosage from time to time when the expected pain relief is delayed.

As reported by Higgins JD et al. (2001) ibuprofen is a weak acid (pKa in the range of 4.5-4.6) and Rivera-Leyva JC et al. (2012) has reported the solubility of Ibuprofen as 0.078 µg/ml (i.e., poorly soluble) in water.

An absolute bioavailability of about 100% defines ibuprofen as "highly permeable", whereas showing pH-dependent solubility (i.e., being insoluble in acidic medium and having an increased solubility only above a pH of 6.5) ibuprofen has been classified a Class II drug according to the present Biopharmaceutical Classification System (BCS). In other words, after oral administration ibuprofen is dissolved and absorbed only in the intestinal tract, but not in the stomach.

Potthast H, Dressman JB, Junginger HE et al. (2005) have issued a biowaiver monograph for immediate release ibuprofen solid oral dasage forms.

Rajanikant P et al. (2010) have expressed that when tablets containing 200 mg of ibuprofen free acid were dissolved in 900 ml of 0.1 N HCl solution at pH 1.2 using USP Type-II (paddle) apparatus rotating at 50 RPM, only 8% of ibuprofen was released in 120 minutes.

It is well documented that ibuprofen salts, such as ibuprofen lysine and ibuprofen arginate, are more rapidly absorbed than formulations of ibuprofen free acid. Another ibuprofen salt, sodium ibuprofen dihydrate has recently been shown to be bioequivalent to the lysine and arginate salt forms.

The chemical structure of ibuprofen sodium dihydrate is shown in Formula IV.

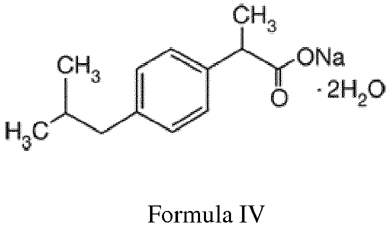

Sörgel F, Fuhr U, Minic M et al. (2005) have studied the comparative in vitro dissolution rates of drug formulations containing ibuprofen sodium dihydrate, ibuprofen free acid, ibuprofen lysine, and ibuprofen arginate in 900 ml of buffer solutions (at pH 1.2, 3.5 and 7.2) at 37°C using USP Type II (paddle) apparatus. The authors concluded that it was possible to reach a 60% dissolution of ibuprofen within 9 minutes at pH 1.2 with a formulation containing ibuprofen sodium dihydrate. The authors also concluded that time to reach maximum plasma concentration (Tₘₐₓ) for ibuprofen sodium dihydrate was significantly earlier than for ibuprofen free acid formulation (Tₘₐₓ: 0.6 ± 0.3 h for ibuprofen sodium dihydrate versus 1.4 ± 1.1 h for ibuprofen free acid).

WO2007/092784 related to the solid ibuprofen concentrate, wherein at least 90% of the weight of the ibuprofen concentrate is ibuprofen free acid and ibuprofen alkali salt. Solid ibuprofen concentrate is obtained by mixing an ibuprofen free acid with a first alkaline substance to form a first composition, then the first composition with a second alkaline substance to form a second composition, and dried. First alkaline substance is selected from the group consisting of a carbonate species, a bicarbonate species, and mixtures thereof.

Although Advil® Allergy & Sinus (Pfizer Consumer Healthcare) contains ibuprofen, pseudoephedrine hydrochloride, and chlorpheniramine maleate, currently there is no commercially available product on the market that comprises a mixture of ibuprofen with ibuprofen sodium dihydrate, together with pseudoephedrine hydrochloride and chlorpheniramine maleate.

In the scope of the present invention, the solubility of ibuprofen versus ibuprofen sodium dihydrate have been studied. Table 1 shows the comparative results when 200 mg of ibuprofen and 256 mg of ibuprofen sodium dihydrate were each dissolved at 250 ml of media having pH of 1.2, pH 4.5, pH 6.8 and 250 ml of water using a magnetic stirrer for 15 minutes.

**TABLE 1**

| | Ibuprofen % | Ibuprofen sodium dihydrate % |
|---|---|---|
| pH 1.2 | 3 | 6 |
| pH 4.5 | 5 | 50 |
| pH 6.8 | 98 | 100 |
| Water | 5 | 100 |

Furthermore, in the scope of the present invention, the dissolution rate of experimental tablets containing 200 mg of ibuprofen free acid have been compared with that of experimental tablets containing a mixture of 175 mg of ibuprofen and 32 mg of ibuprofen sodium dihydrate (equivalent to 25 mg of ibuprofen free acid) (Table 2). The results for the experimental tablets containing 200 mg of ibuprofen free acid have been found compliant with the findings of Rajanikant P et al. (2010), that is only about 8% of ibuprofen released from the experimental tablets containing 200 mg of ibuprofen, whereas more than 15% of said ibuprofen released in 15 minutes when experimental tablets containing 175 mg of ibuprofen free acid and 32 mg of ibuprofen sodium dihydrate (equivalent to 25 mg of ibuprofen free acid) when the tablets are dissolved in 900 ml of 0.1 N HCl solution at pH 1.2 at 37°C using USP Type II (paddle) apparatus rotating at 50 RPM.

**TABLE 2**

| 0.1 N HCl solution at pH 1.2 | Experimental tablets comprising 200 mg of ibuprofen | Experimental tablets comprising 175 mg of ibuprofen and 32 mg of Ibuprofen sodium dihiydrate (= 25 mg ibuprofen free acid) |
|---|---|---|
| 5 min | 4% | 11% |
| 10 min | 6% | 13% |
| 15 min | 8% | 18% |
| 20 min | 10% | 20% |
| 30 min | 12% | 22% |
| 45 min | 15% | 24% |
| 60 min | 16% | 27% |

Incorporation of ibuprofen sodium dihydrate in to the pharmaceutical composition according to the present invention may further enable a much earlier Tmax for ibuprofen and also faster analgesic onset, which is foreseen as one of the biggest advantages of the pharmaceutical composition of the present invention.

The pharmaceutical compositions according to the present invention contain the active ingredients in amounts as indicated in Table 3 below.

**TABLE 3**

| NSAID | Decongestant | Antihistamine |
|---|---|---|
| Ibuprofen + Ibuprofen sodium dihydrate | Pseudoephedrine HCl | Chlorpheniramine maleate |
| Total of 100 mg - 400 mg, when ibuprofen sodium dihydrate is expressed in terms of ibuprofen free acid | 20 mg - 60 mg | 1 mg - 4 mg |

In a preferred embodiment, an oral pharmaceutical composition comprising therapeutically effective amount of:
**a.** ibuprofen and ibuprofen sodium dihydrate mixture;
**b.** pseudoephedrine hydrochloride;
**c.** chlorpheniramine maleate; and
in an acceptable carrier, characterized in that the weight ratio of ibuprofen to ibuprofen sodium dihydrate as free acid equivalent is 19:1 to 1:19 and wherein 15% or more of total ibuprofen dissolves in 15 minutes in 900 ml of 0.1 N HCl solution at pH 1.2 at 37°C using a USP paddle apparatus rotating at 50 RPM with the provision that the ibuprofen and ibuprofen sodium dihydrate mixture does not comprise any inorganic alkaline substances having carbonate, bicarbonate or hydroxide groups.

As the other weight ratios of ibuprofen to ibuprofen sodium dihydrate as free acid equivalent 7:1 to 1:1.

Table 4 shows some of the possible combinations of amounts of ibuprofen and ibuprofen sodium dihydrate, which are not intended to limit the scope of the claimed invention by any manner, for the pharmaceutical composition according to the present invention comprising a total amount of 200 mg of ibuprofen when ibuprofen sodium dihydrate is expressed in terms of ibuprofen free acid, 30 mg of pseudoephedrine hydrochloride, and 2 mg of chlorpheniramine maleate.

**TABLE 4**

| Ibuprofen (A) | Ibuprofen sodium dihydrate (=Ibuprofen free acid =B) | A/B | Pseudoephedrine HCl | Chlorpheniramine maleate |
|---|---|---|---|---|
| 175 mg | 32 mg (=25 mg of ibuprofen free acid) | 7:1 | 30 mg | 2 mg |
| 150 mg | 64 mg (=50 mg of ibuprofen free acid) | 3:1 | 30 mg | 2 mg |
| 125 mg | 96 mg (=75 mg of ibuprofen free acid) | 5:3 | 30 mg | 2 mg |
| 100 mg | 128 mg (=100 mg of ibuprofen free acid) | 1:1 | 30 mg | 2 mg |

The pharmaceutical compositions of the present invention are formulated in a single dosage form for oral administration such as tablet, capsule, powder, granules or a combination thereof.

## Claims

1. An oral pharmaceutical composition comprising:
**a.** 200 mg ibuprofen and ibuprofen sodium dihydrate mixture;
**b.** 30 mg pseudoephedrine hydrochloride;
**c.** 2 mg chlorpheniramine maleate; and
in an acceptable carrier, **characterized in that** the weight ratio of ibuprofen to ibuprofen sodium dihydrate as free acid equivalent is 7:1 to 1:1 and wherein 15% or more of total ibuprofen dissolves in 15 minutes in 900 ml of 0.1N HCl solution at pH 1.2 at 37°C using a USP paddle apparatus rotating at 50 RPM.

2. The oral pharmaceutical composition according to Claim 1, wherein the weight ratio of ibuprofen to ibuprofen sodium dihydrate as free acid equivalent is 7:1.

3. The oral pharmaceutical composition according to Claim 1, wherein the weight ratio of ibuprofen to ibuprofen sodium dihydrate as free acid equivalent is 4:1.

4. The oral pharmaceutical composition according to Claim 1, wherein the weight ratio of ibuprofen to ibuprofen sodium dihydrate as free acid equivalent is 3:1.

5. The oral pharmaceutical composition according to Claim 1, wherein the Based ssss weight ratio of ibuprofen to ibuprofen sodium dihydrate as free acid equivalent is 7:3.

6. The oral pharmaceutical composition according to Claim 1, wherein the weight ratio of ibuprofen to ibuprofen sodium dihydrate as free acid equivalent is 2:1.

7. The oral pharmaceutical composition according to Claim 1, wherein the weight ratio of ibuprofen to ibuprofen sodium dihydrate as free acid equivalent is 5:3.

8. The oral pharmaceutical composition according to Claim 1, wherein the weight ratio of ibuprofen to ibuprofen sodium dihydrate as free acid equivalent is 3:2.

9. The oral pharmaceutical composition according to Claim 1, wherein the weight ratio of ibuprofen to ibuprofen sodium dihydrate as free acid equivalent is 1:1.

10. The oral pharmaceutical composition according to any one of the preceding claims wherein the composition is in the form of a tablet, capsule, powder, granules or a combination thereof.

11. The oral pharmaceutical composition according to Claim 4-2 11, wherein the composition is in the form of a tablet.

12. An oral pharmaceutical composition according to any one of the preceding claims for use for alleviating the symptoms of influenza as well as of common cold, fever, sinusitis, or nasal congestion, headache and general malaise.

## Patentansprüche

1. Orale pharmazeutische Zusammensetzung, umfassend die:
**a)** 200 mg Ibuprofen und Ibuprofen-Natrium-Dihydrat-Mischung;
**b)** 30 mg Pseudoephedrin-Hydrochlorid;
**c)** 2 mg Chlorpheniraminmaleat;und
in einem geeigneten Träger, **dadurch gekennzeichnet, dass** Gewichtsverhältnis von Ibuprofen zu Ibuprofen-Natrium-Dihydrat als freies Säureäquivalent 7:1 bis 1:1 beträgt und wobei 15% oder mehr des gesamten Ibuprofens sich in 15 Minuten in 900 ml einer 0.1 N HCl-Lösung auflöst bei einem pH-Wert von 1.2 bei 37°C unter Verwendung einer USP-Paddel-Apparatus, die bei 50 U / Minute rotiert.

2. Orale pharmazeutische Zusammensetzung nach Anspruch 1, wobei das Gewichtsverhältnis vonIbuprofen zu Ibuprofen-Natrium-Dihydrat als Äquivalent der freien Säure 7:1 beträgt.

3. Orale pharmazeutische Zusammensetzung nach Anspruch 1, wobei das Gewichtsverhältnis von Ibuprofen zu Ibuprofen-Natrium-Dihydrat als Äquivalent der freien Säure 4:1 beträgt.

4. Orale pharmazeutische Zusammensetzung nach Anspruch 1, wobei das Gewichtsverhältnis vonIbuprofen zu Ibuprofen-Natrium-Dihydrat als Äquivalent der freien Säure 3:1 beträgt.

5. Orale pharmazeutische Zusammensetzung nach Anspruch 1, wobei die basierte SSSS-Gewichtsverhältnis von Ibuprofen zu Ibuprofen-Natrium-Dihydrat als freies Säureäquivalent 7:3 beträgt.

6. Orale pharmazeutische Zusammensetzung nach Anspruch 1, wobei das GewichtsverhältnisvonIbuprofen zu Ibuprofen-Natrium-Dihydrat als Äquivalent der freien Säure 2:1 beträgt.

7. Orale pharmazeutische Zusammensetzung nach Anspruch 1, wobei das Gewichtsverhältnisvon Ibuprofen zu Ibuprofen-Natrium-Dihydrat als freies Säureäquivalent 5:3 beträgt.

8. Orale pharmazeutische Zusammensetzung nach Anspruch 1, wobei das Gewichtsverhältnisvon Ibuprofen zu Ibuprofen-Natrium-Dihydrat als Äquivalent der freien Säure 3:2 beträgt.

9. Orale pharmazeutische Zusammensetzung nach Anspruch 1, wobei das Gewichtsverhältnisvon Ibuprofen zu Ibuprofen-Natrium-Dihydrat als freies Säureäquivalent 1:1 beträgt.

10. Orale pharmazeutische Zusammensetzung nach einem der vorhergehenden Ansprüche, wobeidie Zusammensetzung in Form einer Tablette, Kapsel, Pulver, Granulate oder einerKombination vorliegt.

11. Orale pharmazeutische Zusammensetzung nach Anspruch 10, wobei die Zusammensetzung in Form einer Tablette vorliegt.

12. Orale pharmazeutische Zusammensetzung nach einem der vorhergehenden Ansprüche zur Verwendung zur Linderung der Symptome von Influenza sowie von Erkältung, Fieber, Sinusitis oder verstopfter Nase. Kopfschmerzen und allgemeine Unwohlheiten.

## Revendications

1. Une composition pharmaceutique orale comprenant, dans un support acceptable :
a) 200 mg d'un mélange d'ibuprofène et d'ibuprofène sodique dihydraté
b) 30 mg de chlorhydrate de pseudoéphédrine;
c) 2 mg de maléate de chlorphéniramine; et
**caractérisée en ce que** le rapport pondéral entre l'ibuprofène et l'ibuprofène sodique dihydraté en équivalent d'acide libre est de 7:1 à 1:1 et dans lequel 15% ou plus de l'ibuprofène total se dissout en 15 minutes dans 900 ml d'une solution de HCl 0,1 N à pH 1.2 à 37°C en utilisant un appareil à palettes USP tournant à 50 tr / min.

2. Composition pharmaceutique orale selon la revendication1, dans laquelle le rapport pondéral entre l'ibuprofène et l'ibuprofène sodique dihydraté en équivalent d'acide libre est de 7:1.

3. Composition pharmaceutique orale selon la revendication 1, dans laquelle le rapport pondéral entre l'ibuprofène et l'ibuprofène sodique dihydraté en équivalent d'acide libre est de4:1.

4. Composition pharmaceutique orale selon la revendication 1, dans laquelle le rapport pondéral entre l'ibuprofène et l'ibuprofène sodique dihydraté en équivalent d'acide libre est de3:1.

5. Composition pharmaceutique orale selon la revendication 1, dans laquelle le rapport pondéral entre l'ibuprofène et l'ibuprofène sodique dihydraté en équivalent d'acide libre est de7:3.

6. Composition pharmaceutique orale selon la revendication 1, dans laquelle le rapport pondéral entre l'ibuprofène et l'ibuprofène sodique dihydraté en équivalent d'acide libre est de2:1.

7. Composition pharmaceutique orale selon la revendication 1, dans laquelle le rapport pondéral entre l'ibuprofène et l'ibuprofène sodique dihydraté en équivalent d'acide libre est de5:3.

8. Composition pharmaceutique orale selon la revendication 1, dans laquelle le rapport pondéral entre l'ibuprofène et l'ibuprofène sodique dihydraté en équivalent d'acide libre est de3:2.

9. Composition pharmaceutique orale selon la revendication 1, dans laquelle le rapport pondéral entre l'ibuprofène et l'ibuprofène sodique dihydraté en équivalent d'acide libre est de1:1.

10. Composition pharmaceutique orale selon l'une des revendications précédentes, dans laquelle la composition se présente sous la forme d'un comprimé, d'une capsule, d'une poudre, de granulés ou d'une combinaison de ceux-ci.

11. Composition pharmaceutique orale selon la revendication 11, dans laquelle la composition est sous la forme d'un comprimé.

12. Composition pharmaceutique orale selon l'une des revendications précédentes, destinée à être utilisée pour soulager les symptômes de la grippe ainsi que du rhume, de la fièvre, de la sinusite ou de la congestion nasale, des maux de tête et d'un malaise général.
